# EUROPEAN PATENT APPLICATION

(11) **EP 3 835 029 A1**
(43) Date of publication of application: **16.06.2021**
(21) Application number: 19215495.3
(22) Date of filing: 12.12.2019
(51) Int. Cl.: B29C 64/00, B60H 3/00, A61L 9/00, B33Y 80/00, B33Y 70/10, B29C 64/10

(54) **METHOD AND DEVICE FOR PROVIDING OR ABSORBING A SCENT**

(71) Applicant: Bayerische Motoren Werke Aktiengesellschaft, 80809 München (DE)
(72) Inventor: Iliffe-Moon, Etienne, Menlo Park, 94025 (US)

(57) **Abstract**

Disclosed is a method, comprising the steps:
- forming a matrix by an additive manufacturing process;
- providing a scent material and/or an absorber material to the matrix during and/or after the additive manufacturing process.

## Description

### Technical field

In this disclosure embodiments are described which relate to methods for manufacturing a device for providing a scent to an environment and/or for absorbing a scent from an environment and which relate to devices for providing and/or manufacturing a scent to/from an environment, in particular to/from a passenger cabin of a vehicle.

### Background

Known fragrance dispensers, for example for passenger cars or living rooms, are designed in the form of containers which are clipped onto the ventilation grilles and which contain a liquid substrate to be dispensed into the intended space. Furthermore, there are fragrance dispensers in the form of granules for an ashtray or as a "fragrance tree" configured to hang from the rear-view mirror. Other systems comprise one of more fragrances that are stored in a compartment or cartridge of the system and further comprise a motor in order to provide air charged with the respective fragrance into a passenger cabin or a living room. Further devices allow for fragrances to be atomized and then distributed as evenly as possible in large rooms by means of a stream of air through ventilation or air conditioning systems. The atomization can be carried out by means of an ultrasonic generator, a sound transducer with sonotrode and a suitable media supply. In general, scent can be either in a liquid or solid format. A solid fragrance format has benefits, in particular for automotive applications, because liquid scent delivery can be messy and the delivery devices can become clogged, e.g. spray nozzles. Furthermore, a solid format can be more robust in presence of higher temperatures. While solid formats are more convenient with respect to usage, liquid formats provide advantages when it comes to re-fill or dispersion of the fragrance.

### Description

An object of the present invention is to provide an improved technology for providing a scent or a fragrance to an environment and/or for absorbing a scent or a fragrance from an environment.

This problem is solved by the invention, which is defined by the subject matter of the independent claims. The dependent claims relate to corresponding embodiments. In the following, different aspects and embodiments of these aspects are disclosed, which provide additional features and advantages.

Some embodiments of the present disclosure provide a 3D-printed polymer matrix with a maximized surface in order to provide a scent into a passenger cabin of a vehicle, such as a car, a train, or an airplane.

A first aspect relates to a method, comprising the steps:
- forming a matrix by an additive manufacturing process;
- providing a scent material and/or an absorber material to the matrix during and/or after the additive manufacturing process.

The method can be used for manufacturing a device for providing a scent to an environment and/or for absorbing a scent from an environment.

A scent can be an odor, a fragrant, an aroma, a perfume, a smell and/or any combination thereof. It can comprise any material that can be olfactorily sensed by a human and/or an animal.

An additive manufacturing process within the meaning of this disclosure can comprise in particular stereolithography, laser beam melting, laser sintering, layer laminated manufacturing, electron beam melting, fused layer modelling, multi jet modelling, poly jet modelling, digital light processing, thermal transfer sintering and/or 3D printing, e.g. extrusion printing. An additive manufacturing process can further comprise a photopolymerization, material or binder jetting, powder fusion, material extrusion, and/or sheet lamination.

An environment in the meaning of this disclosure can be any space that is used and/or occupied by a human being and/or an animal. In particular, an environment can be a passenger cabin of a vehicle, such as a car, boat and/or an airplane. Further, an environment can be a room of a house, in particular a bathroom or a living room.

A matrix can be any material or composite that is configured to comprise another material, such as a scent material or an absorber material. Thereby, the sent material and/or the absorber material act as the functional filler of the matrix. Different kinds of matrices can be possible. For example, a matrix can be an organic matrix, such as a polymer. Furthermore, a matrix can comprise a ceramic matrix and/or a metal matrix. In particular, a matrix can be reinforced by fiber materials, in particular to achieve a larger structural longevity and/or a predefined mechanical characteristic, for example a pre-defined stiffness. Additionally or alternatively, a matrix can be a paper or a similar structure and in particular filled with a starch-based material. The starch-based material will then comprise a sent media and/or an absorber material.

The scent material and/or the absorber material can be a liquid material, a semi-liquid material or a solid material, in the presence of room temperature. The scent material and/or the absorber material can also be a gel. A scent material can be in particular a material that carries a scent and which is deluded with a solvent for example an alcohol, water and/or a rectified spirit, e.g. an isopropyl myristate. Furthermore, the scent material can be diluted by fractionated coconut oil, liquid waxes, in particular Jojoba oil.

An embodiment of the first aspect relates to a method, wherein the matrix is formed such that it comprises a polymer.

A matrix can comprise in particular a polymer with an organic filler. The organic filler can for example be a coconut-basedfiber filler material. The filler is combined with the polymer such that the 3D printed polymer matrix can absorb the liquid and later set the liquid free to provide a certain kind of scent to and environment. In other words, the 3D print material can be a combination of a polymer, which acts as a binder, with a filler, e.g. coconut derived fibers, which is 3D printed as a single material into the desired structure.

An embodiment of the first aspect relates to a method, wherein the matrix is formed such that it comprises an absorbent material, being a hydrophilic and/or a hydroscopic material or being similar to a hydrophilic and/or a hydroscopic material that has the propensity to absorb whatever the fragrance material is, e.g. being able to absorb an alcohol-based material and/or being able to absorb a water-based material.

A hygroscopic material is a material that absorbs moisture at a large rate. Hygroscopic substances include cellulose fibers, such as cotton and/paper, sugar, caramel, honey, glycerol, ethanol, wood, methanol, sulfuric acids, salts, such as calcium chloride and/or sodium hydroxide, for example. Hydrophilic substances can comprise zinc chloride and calcium chloride, potassium hydroxide, sodium hydroxide, and/or salts. In particular hygroscopic and/or hydrophilic materials can be used for an absorber material, which is used as a functional filler of a matrix. The matrix can be configured such that a scent material and/or an absorber material can be infused and/or absorbed during and/or after the additive manufacturing step, in particular during and/or after a 3D-printing. This can be done by one or more of the above-named features and/or by one of the features described in the remainder. Materials used for the matrix and/or for the absorber material can be superabsorbent. In particular a matrix can be absorbent and superabsorbent, such that it can store and provide a fragrance to the environment and afterwards clean the environment by absorbing a scent.

An embodiment of the first aspect relates to a method, wherein the matrix is formed to comprise a lattice structure.

A lattice structure can comprise an open cellular structure, equal or similar to a reticulated foam. Reticulated means that the foam has a network-like design. A reticulated foam is a porous, low density solid foam, such that it will allow the passage of fluids, e.g. such as air or liquids, through the matrix. The solid component of a reticulated foam may be an organic polymer like polyurethane, a ceramic or a metal. A reticulated foam or a material similar to a reticulated foam can be produced through a gas release process, such that the foam structure is basically a random structure. Additionally or alternatively, a reticulated foam or a material similar to a reticulated foam can have pre-defined geometric properties, in particular by a CAD-design process step conducted in an early step of the method. Furthermore, a lattice structure can also comprise a form that is not reticulated. Advantageously, an open cell structure material provides a large surface to provide a scent material to an environment and/or to absorb a scent from an environment. Furthermore, an open cell structure can be advantageously used as a matrix to host a filler material, in particular a scent and/or an absorber material.

A lattice can be designed through conventional 3D CAD tools or by use of generative and/or algorithmic 3D CAD-tools in a prior method step. The lattice structure can be randomly organized or structured and/or geometrically patterned, for example by a repeated geometry that follows a pre-defined pattern.

An embodiment of the first aspect relates to a method, wherein the matrix is formed to comprise a tree structure.

A tree structure can in particular comprise a trunk, from which branches extend. Additionally or alternatively, a tree structure can comprise leaves that extend from a trunk and/or a branch of a trunk. Advantageously, the tree structure can be designed to optimize airflow and provide a large surface in order to diffuse a scent material and/or to absorb a scent from an environment. In particular, the ratio of the surface area of the device, in particular of the matrix, to its 3D-volume can be optimized for absorption, deabsorption and/or internal diffusion. A tree structure in the sense of this disclosure does of course not comprise the typical structure of a "fragrance tree". A tree structure might have one or more structural main features, for example columns, from which geometric sub-features are supported. For example, the main features and sub-features could be linear, radial, helical and/or a combination of several formats. A design of the device and/or the matrix for airflow can be optimized by using Computer Fluid Dynamics and/or using Generative 3D CAD tools to computationally pre-define the external and/or internal structure of the device, in particular of the matrix, in an early step of the method.

An embodiment of the first aspect relates to a method, wherein the matrix is formed to comprise one or more cavities.

In particular a cavity can be an open cavity. An open cavity advantageously provides an increased surface that can be used for diffusing a scent material and/or absorbing a scent from air that flows through and/or within the open cavity. Additionally or alternatively, a matrix can be formed such that it comprises one or more closed cavities. One or more closed cavities can in particular be used as container for storing a scent material and/or an absorber material, for example a cavity can be used for a cartridge and/or a section of a cartridge. In particular closed cavities can be formed of porous material such that a scent material can evaporate to the outside and/or that scented air can evaporate into the cavity, such that the scent can be absorbed by an absorber material contained in the cavity. In particular, closed and/or open cavities can be formed by a 3D-printing process, for example cavities that could otherwise not be produced with conventional manufacturing techniques.

In particular the device and/or the matrix can be designed, by a lattice-, cavity- and/or tree structure such that an airflow is directed into a predefined direction, in particular when passing through the matrix. In particular this can be done by 3D-printing a geometry into the device, in particular into the matrix, that performs additional functions like guiding and/or funneling of the flow of air, creating a venturi to control the air speed and/or pressure, or by one or more features to control any excess or seepage of scent material, e.g. by valves. To achieve this object a feature to attach the device within the environment may correspond to the structure of the device and/or the matrix in order that an airflow is directed into a pre-defined direction.

An embodiment of the first aspect relates to a method, wherein the matrix is provided with a scent and wherein the matrix is formed such that a scent is diffused at an essentially constant rate.

An essentially constant diffusion rate of a scent can be in particular implemented by a matrix that contains a scent material and wherein an evaporation rate of the scent material is equal, or at least similar, to a supply rate by which the scent material is supplied to the surface of the matrix to evaporate into the environment. To match the evaporation rate of a matrix by the supply rate, the matrix can be configured to have a very high flow rate by which a scent can move from a supply to an outer surface of the matrix. Alternatively or additionally, a supply at a high supply rate of liquid might not be necessary, if the matrix is itself the container for the scent material. Therefore, the matrix can comprise internal cavities and/or an open cell structure such that the scent material can be stored throughout the whole matrix. Furthermore, the matrix can be used comprising a lattice structure and wherein the lattice structure in itself is built out of a lattice structure, comprising internal cavities to supply a scent material and/or an absorber material.

In a further embodiment, the device, in particular the matrix, is formed such that a consistency of fragrance release from the scent material, over the life of the scent material is improved, in particular maximized. This can be done in particular by controlling the surface area of the device exposed to the airflow. Additionally or alternatively, this can be done by predefining a composition of 3D print material, e.g. a type of material and/or polymer, to control the absorption and desorption and/or diffusion of the scent material. Additionally or alternatively, this can be done by a hybridizing material, in particular with a secondary filler material that is an absorbent, e.g. an organic material, and/or aids the diffusion of the scent material.
Additionally or alternatively, this can be done by pre-defining a geometric form of the device and/or the matrix, in particular to comprise one or more open areas and/or a pre-defined density. Additionally or alternatively, this can be done by selecting pre-defined cross-sections of structural elements, in particular of the matrix, to control the diffusion distance to the surface of the structure. A further alternative to increase a consistency of fragrance release can be to vary a homogeneity of the scent material and/or of the material of the matrix, for example by printing voids and/or air bubbles in the material on a small scale. For example this can be done such that the overall structure of the matrix has an open cell form, with cells of e.g. 3 mm diameter, and which comprises a further structure that has a cell form with diameters smaller than 1 mm, which are open or closed. Additionally or alternatively, this can be done by controlling the composition of a scent material, e.g. alcohol-based fragrances diffuse at a larger rate than water-based fragrances. Additionally or alternatively, this could be done by providing features to control the airflow, release of scent and/or rate of diffusion, which is described later in more detail.

In this disclosure "absorption" means an ability of a material, or a structure, e.g. the matrix, to absorb the scent material or an external odor. Furthermore, "internal diffusion" means diffusion of a scent material within the matrix material. The term "evaporation" means the release of scent from the matrix surface into the environment, in particular an airflow stream. The term "airborne diffusion" means diffusion of scent (after evaporation from the matrix surface) in the ambient air.

An embodiment of the first aspect relates to a method, wherein the matrix is formed such that it comprises a cartridge configured to store and/or to provide the scent material and/or the absorber material.

In particular the cartridge can comprise multiple containers in order to provide different kinds of scent material or a scent material to be diffused according to different parameters. Such parameters can be in particular a time and/or a signal, in particular signal coming from a person that uses the device. Additionally or alternatively, the cartridge can be configured to hold one or more absorber materials. Also, for the absorber materials, the cartridge can be configured such that the absorber function of the device is activated based on a predefined parameter, such as time and/or signal input from a user. In particular the device can be formed such that it contains a scent material and/or an absorber material and provides the same material at different times, for example at the beginning of a use an absorber function is activated to clean an environment from a certain scent and afterwards and scent function is activated in order to provide a predefined scent contained in the device to the environment. Thereafter, in particular an absorber function can be activated again in order to clean the environment from the scent provided in the step before. The scent material and/or the absorber material can be provided in a different step then the matrix, in particular also by an additive manufacturing step, such that the material can be provided to the matrix and replaced after it has been used.

An embodiment of the first aspect relates to a method, wherein the matrix is provided with an absorber material that comprises carbon, baking soda and/or betacyclodextrin.

Other scent absorbing materials can be based or comprise vinegar, citrus, baking soda, coffee grounds, and charcoal. By the additive manufacturing process one or more absorber materials can be provided in particular directly into the matrix and/or as a structure to be inserted into a container of the matrix and/or a supply that can be connected to the device and/or the matrix.

An embodiment of the first aspect relates to a method, comprising the step:
- forming a feature for attaching the device to an environment and/or to a scent delivery device.

In particular the matrix can be formed such it comprises the feature for attaching the device to an environment. The feature can be formed such that the device is arranged in the vicinity of a fan or an air conditioning system. Advantageously, thereby an airflow can be used from these devices in order to guide air over or through the device to be enriched with a scent material and/or in order to scent of the air being absorbed by the device. A scent delivery device can be an electromechanical device to digitally control the release of scent, in particular on the basis of data, for example sensor data, from a car or a smart device, e.g. a smart phone.

An embodiment of the first aspect relates to a method, comprising the step:
- forming an interface to the device for interfacing the matrix to a scent-material-and/or an absorber-material-delivery-hardware.

In particular the feature can be formed into the matrix during the additive manufacturing step.

An interface can comprise an interface for providing and/or setting off a scent material and/or an absorber material in a liquid, gaseous, and/or solid material. In particular the interface can also be combined with a mechanical interface to arrange the device at a location within an environment, such as a passenger cabin.

An embodiment of the first aspect relates to a method, comprising the step:
- forming a feature for attaching the device to a fan blower and/or to and/or within an air conditioning system.

In particular the feature can be formed into the matrix during the additive manufacturing step. Furthermore, the attachment can be configured to mount the device within or next to an air conditioning system. Additionally or alternatively, the device can be formed such that it comprises one or more features to guide an airstream from a fan blower and/or an air conditioning system. A feature to guide or funnel airflow can be in particular one or more of a wall, a channel, a venturi, a valve, a gear and/or a linkage. Thereby, the device can be configured to control an airflow, a release of scent and/or a rate of diffusion. In particular, one or more of these features can be provided within the additive manufacturing step by which the matrix is formed. Alternatively or additionally, one or more of these features could be formed during an additional step, in particular also by an additive manufacturing such as 3D-printing. In particular the matrix can comprise such a feature.

An embodiment of the first aspect relates to a method, wherein the matrix is formed into a pre-existing form, in particular a pre-existing cartridge.

By this, the pre-existing cartridge can be re-used.

An embodiment of the first aspect relates to a method, wherein the matrix and at least one of the scent materials and the absorber material is formed such that they can be assembled in a subsequent step of the method.

In a further embodiment the device, in particular the matrix, is formed such that a scent material and/or an absorber material can be re-filled, in particular after usage or if the scent and/or absorber material should be changed.

A second aspect relates to a device, comprising:
a matrix comprising a lattice structure, a tree structure and/or one or more cavities;
a scent material and/or an absorber material as a functional filler of the matrix.

The device can be used for providing a scent to an environment and/or for absorbing a scent from an environment.

The matrix can have in particular a linear, a helical and/or radial structure. Furthermore, the device can have one or more functions and/or features as described within the context of first aspect.

### Short Description of the Figures

Further advantages and features result from the following embodiments, which refer to the figures. The figures describe the embodiments in principle and not to scale. The dimensions of the various features may be enlarged or reduced, in particular to facilitate an understanding of the described technology. For this purpose, it is shown, partly schematized, in:
Fig. 1 a device with a tree structure according to an embodiment of the disclosure;
Fig. 2 a device with a lattice structure according to an embodiment of the disclosure.

In the following descriptions, identical reference signs refer to identical or at least functionally or structurally similar features.

In the following description reference is made to the accompanying figures which form part of the disclosure and which illustrate specific aspects in which the present disclosure can be understood.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Description of the Figures

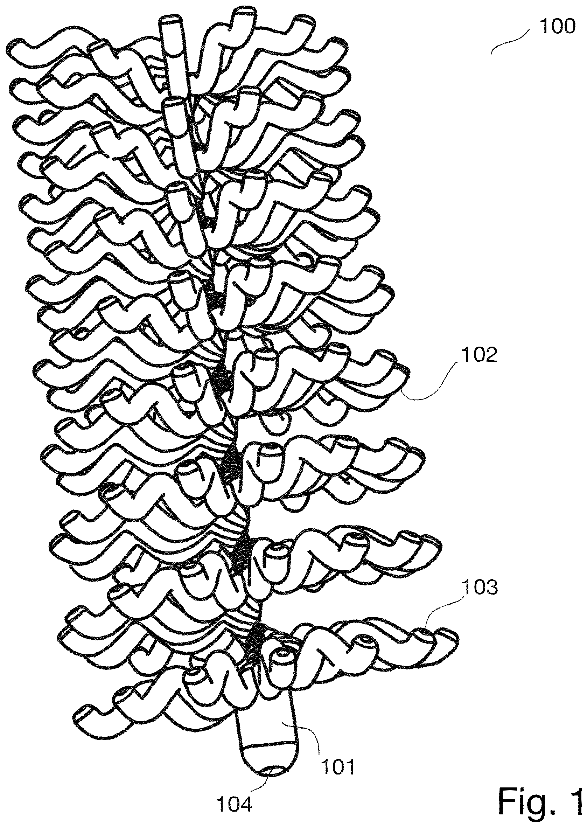

Fig. 1 shows a device 100 for providing a scent to the environment and/or for absorbing a scent from an environment. The device predominantly consists of a matrix combined with a scent and/or absorber material. The device is 3D-printed, in particular by a 3D-printer with different printing heads. Thereby, one printing head can provide the material for the matrix and a further printing head can provide a scent material (not shown) and/or an absorber material to be integrated into the matrix. Additionally or alternatively, a filament can be generated with a combination of a polymer and a fragrance material, such that the filament loaded into the 3D printer and with which the device, in particular the matrix is printed, is pre-scented. It comprises a trunk 101 from which a plurality of branches 102 extend. The device is formed with internal channels and cavities (not displayed). Further, the device is formed out of a porous material in order to facilitate evaporation of scent material into the environment and/or in order to facilitate an airflow through the device in order to absorb a scent from the air flow. Further scent material and/or absorber material can be provided to the device 100 by the interface 104 at the bottom of the trunk of the device. Furthermore, the interface 104 can also be used to mount the device to an environment, in particular in the vicinity of an air intake of a vehicle and/or of an air conditioning. From the interface 104 channels are formed within the device that lead to the interface 103, which is formed at the end of each branch. Through the interface 103 air can stream into and/or out of the device. The channels connect the interface 104 with each of the interfaces 103, which are located at the end of each branch. The channels are used to deliver a scent material to the surface of the device 100. By the plurality of branches, the device 100 exhibits a large surface compared to its volume. Additionally or alternatively, a surface-area-to-volume-ratio can be optimized for characteristics of a scent material, a material of the matrix and/or a desired rate of evaporation, in particular in order to achieve a desired fragrance intensity and/or life of the scent material, in particular a pre-defined average diffusion rate. This large surface facilitates an evaporation of a scent material to an environment or vice versa. Of course, other forms are possible that also provide a large surface compared to the volume of the device, for example lattice structures and/or open cell structures. They could be in principle designed as the tree structure 100.

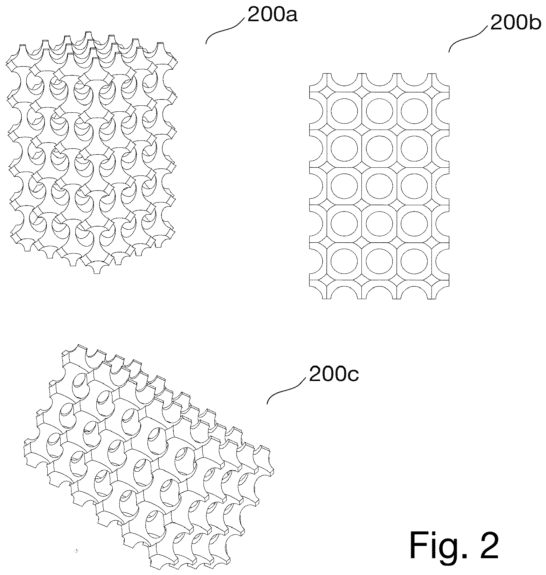

Fig. 2 shows a device with a lattice structure according to an embodiment of the disclosure. The lattice structure in Fig. 2 is depicted in two perspective views 200a, 200c and a side view 200b. It is structured by a CAD-design process prior to 3D-printing and shows a matrix with a geometrical pattern in order charge an airflow with a scent material being stored in the matrix.

### List of Reference Signs

- 100: device according to an embodiment of the disclosure
- 101: trunk
- 102: branch
- 103: interface to a channel within the device
- 104: interface to a supply for a scent material
- 200a: perspective view of a device according to an embodiment of the disclosure
- 200b: perspective view of a device according to an embodiment of the disclosure
- 200c: side view of a device according to an embodiment of the disclosure

## Claims

1. A method for manufacturing a device (100) for providing a scent to an environment and/or for absorbing a scent and/or an odor from an environment, comprising the steps:
- forming a matrix by an additive manufacturing process;
- providing a scent material and/or an absorber material to the matrix and/or to the material by which the matrix is formed before, during and/or after the additive manufacturing process.

2. The method of claim 1, wherein the matrix is formed such that it comprises a polymer.

3. The method according to one of the preceding claims, wherein the matrix is formed such that it absorbs the scent material and/or a scent and/or an odor from an environment, in particular by a hydrophilic and/or a hydroscopic material.

4. The method according to one of the preceding claims, wherein the matrix is formed to comprise a lattice structure.

5. The method according to one of the preceding claims, wherein the matrix is formed to comprise a tree structure (101, 102).

6. The method according to one of the preceding claims, wherein the matrix is formed to comprise one or more cavities (103, 104).

7. The method according to one of the preceding claims, wherein the matrix is provided with a scent and wherein the matrix is formed such that a scent is diffused at an essentially constant rate.

8. The method according to one of the preceding claims, wherein the matrix is formed such that it comprises a cartridge configured to store and/or to provide the scent material and/or the absorber material.

9. The method according to one of the preceding claims, wherein the matrix is provided with an absorber material that comprises carbon, baking soda and/or betacyclodextrin.

10. The method according to one of the preceding claims, comprising the step:
- forming a feature (104) for attaching the device (100) to an environment.

11. The method according to one of the preceding claims, comprising the step:
- forming an interface (104) to the device for interfacing the matrix to a scent-material-and/or an absorber-material-delivery-hardware.

12. The method according to one of the preceding claims, comprising the step:
- forming a feature for attaching the device (100) to a fan blower and/or to and/or within an air conditioning system, and wherein in particular the device is formed to guide an air stream from a fan blower and/or an air conditioning system.

13. The method according to one of the preceding claims, wherein the matrix is formed into a pre-existing form, in particular a pre-existing cartridge.

14. The method according to one of the preceding claims, wherein the matrix and at least one of the scent material and the absorber material is formed such that they can be assembled in a subsequent step of the method.

15. A device (100) for providing and/or absorbing a scent, comprising:
a matrix comprising a lattice structure, a tree structure and/or one or more cavities;
a scent material and/or an absorber material as a functional filler of the matrix (100).
